# EUROPEAN PATENT SPECIFICATION

(11) **EP 1 604 652 B1**
(45) Date of publication and mention of the grant of the patent: **14.01.2009**
(21) Application number: 05104956.7
(22) Date of filing: 07.06.2005
(51) Int. Cl.: A61K 9/70, A61K 9/22, A61K 9/00

(54) **Topical anaesthetic preparation for pretreatment for upper gastrointestinal endoscopy**
Topische anesthetische Zubereitung zur Vorbehandlung von oberer gastrointestinaler Endoscopie
Préparation topique anésthésique pour le prétraitement en endoscopie gastrointestinale supérieure

(30) Priority: 07.06.2004 JP 2004169181
(43) Date of publication of application: 14.12.2005
(73) Proprietor: NITTO DENKO CORPORATION, Ibaraki-shi, Osaka 567-8680 (JP)
(72) Inventor: Saito, Junichi, 567-8680, Osaka (JP); Ninomiya, Kazuhisa, 567-8680, Osaka (JP)
(74) Representative: von Kreisler Selting Werner

(56) References cited:
- EP-A2- 1 181 931
- WO-A-95/01788
- WO-A-96/21432
- WO-A2-98/55079

## Description

### FIELD OF THE INVENTION

The present invention relates to a topical anesthetic preparation according to claim 1 for a pretreatment for upper gastrointestinal endoscopy. This topical anesthetic preparation is used for suppressing the pharyngeal reflex caused by insertion of an endoscope into the pharynx.

### BACKGROUND OF THE INVENTION

To examine the stomach or the esophagus primarily for formation of a tumor, upper gastrointestinal endoscopy has been widely used in medical institutions and so on. In upper gastrointestinal endoscopy, test subjects most often feel pain due to the pharyngeal reflex caused by insertion of an endoscope into the pharynx. To suppress the pharyngeal reflex, it is a general practice to anesthetize the pharynx by making test subjects contain a topical anesthetic viscous agent in a mouth cavity for a given time immediately before endoscopy.

The topical anesthetic viscous agent contains a flavoring and a sweetener. Nevertheless, this anesthetic tastes extremely bitter. As a result, the test subject under unbearable bitterness may spit out the topical anesthetic viscous agent in the mouth before sufficient anesthetic effect is obtained.

It is sometimes difficult to determine from the outside if the topical anesthetic viscous agent is contained in the mouth cavity, because the topical anesthetic viscous agent in itself is not very big. The topical anesthetic viscous agent is colorless and transparent. Therefore, when the test subject is an individual difficult to communicate with, such as a child and the like, and the individual does not contain the topical anesthetic viscous agent in his or her mouth cavity, it is difficult to determine if it is not in the mouth cavity because the anesthetic has not been administered or because the anesthetic has accidentally been ingested.

When in use, the topical anesthetic viscous agent is contained in the mouth cavity for several minutes and then spit out. This means that only a part of a dose of the topical anesthetic viscous agent is used as a topical anesthetic and most part thereof is spit out. Accordingly, the utilization rate of the topical anesthetic is not very high.

As topical anesthetics administered by a method other than containing in the mouth cavity for a given time as adopted for a topical anesthetic viscous agent, a method of spraying a topical anesthetic spray directly to the pharynx can be mentioned. However, the operation of spraying is complicated. In addition, infection with a disease is feared by sharing a topical anesthetic spray with plural test subjects.

As a different method, a method comprising anesthetizing and relieving pain from the throat with inflammation by the use of an anesthetic-containing chewing gum composition comprising a chewing gum base containing an anesthetic has been proposed (USP 4,822,597).

To achieve an effect by the use of an anesthetic-containing chewing gum composition, it is necessary to chew for a certain time. In the case of a toothache and the like due to inflammation in the mouth cavity, absence of teeth and the like, therefore, the effect is difficult to achieve.

Besides these, as topical anesthetic preparations to be used in the mouth cavity, a mucous membrane adhesion preparation comprising a mucous membrane adhesive layer containing an alginic acid ester and a pharmaceutical agent formed on a substrate (JP-A-61-30516), a gingiva application type topical anesthetic adhesive agent containing a physiologically active component in an adhesive layer containing a water-soluble polymer such as polyvinylpyrrolidone etc. and a water insoluble or water swellable polymer such as vinyl acetate etc. (JP-A-1-272521), an intraoral application substrate having a two-layer membrane obtained by casting and drying a liquid containing a polyacrylic acid salt, a carboxyvinyl polymer and polyvinyl alcohol (JP-A-5-310561), a self-adhesive oral transmucosal drug administration agent (JP-T-2002-531393) and the like have been proposed. However, these are either inapplicable for a pretreatment for endoscopy or insufficient in the required properties.

EP-A-1181931 discloses an intra oral adhesive preparation, which has a support made of a cloth and a pressure-sensitive adhesive layer containing a drug. WO/9501788 deals with an article for delivering a drug to the oral cavity; the article can be a brush.

### SUMMARY OF THE INVENTION

As mentioned above, a topical anesthetic preparation usable for a pretreatment for upper gastrointestinal endoscopy, which has a high drug utilization rate, and preferably, which permits easy identification of use has not been provided. It is therefore a problem of the present invention to provide such a topical anesthetic preparation for a pretreatment for upper gastrointestinal endoscopy.

The present inventors have conducted intensive studies and completed a topical anesthetic preparation that accomplishes the above-mentioned object.

The topical anesthetic preparation of the present invention is used for a pretreatment for upper gastrointestinal endoscopy. According to the present invention, since a plaster layer directly comes into contact with the action site in the oral cavity, a topical anesthetic can be used highly efficiently. Since the topical anesthetic preparation of the present invention has a support having a particular stiffness, the test subjects do not feel pain very often. When the topical anesthetic preparation of the present invention has a gripper, whether or not the preparation is being used can be known easily.

### BRIEF DESCRIPTION OF THE DRAWINGS

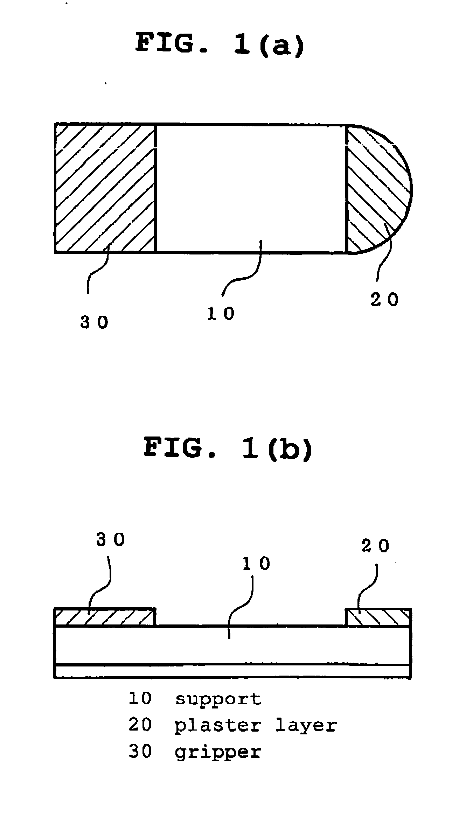

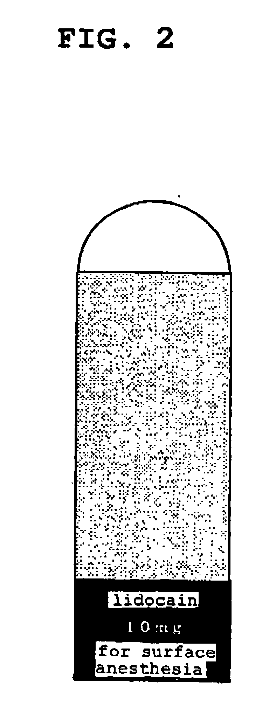

Fig. 1(a) is a plane view of one embodiment of the topical anesthetic preparation of the present invention and Fig. 1(b) is a side view thereof. Fig. 2 is a plane view of one embodiment of the topical anesthetic preparation of the present invention. In the Figures, 10 is a support, 20 is a plaster layer and 30 is a gripper.

### DETAILED DESCRIPTION OF THE INVENTION

The topical anesthetic preparation of the present invention has a support, a plaster layer and a gripper. The topical anesthetic preparation of the present invention is intraorally used for a pretreatment for upper gastrointestinal endoscopy. The upper gastrointestinal tract includes esophagus, stomach, duodenum and jejunum.

Fig. 1(a) is a plane view of one embodiment of the topical anesthetic preparation of the present invention and Fig. 1(b) is a side view thereof. In Fig. 1, each constitution is exaggerated to facilitate understanding of the outline of the construction of the present invention. In effect, the gripper and the like are thinner than depicted and the support is longer than shown in the longitudinal direction.

As shown in Fig. 1, the topical anesthetic preparation of the present invention has a long support 10. The support 10 has a plaster layer 20 containing at least a topical anesthetic and an adhesive polymer on one surface or both surfaces of one end in the longitudinal direction. Preferably, a gripper 30 is formed on the other end of the support 10 in the longitudinal direction.

The support 10 needs to have strength in a particular range, When the strength is too small, one end of the topical anesthetic preparation of the present invention cannot be easily reached to near the base of the tongue that should be topically anesthetized during use, along the curve of the oral cavity of the test subject. When the strength is too high, the preparation gives pain to the test subject. In the present invention, the strength of the support is specified by the stiffness described below.

The stiffness is determined by Method A (45° cantilever method) or Method B (Gurley method) as defined in the JIS standard, non-woven fabric interlining test method (L1085¹⁹⁹²), 5.7 stiffness. The stiffness shows the level of resistance to the bending deformation, and is one of the evaluation indices of flexibility of cloths and films. The 45° cantilever method is performed using a test apparatus having the shape of a cantilever. A 2 cmx15 cm test piece is placed on a horizontal table of the test apparatus with a short side fitting the scale base line. The horizontal table has a smooth surface and a slope with an angle of 45° on one end. Then, the test piece is allowed to slide gently in the direction of the inclined plane, and when one end of the test piece contacts the inclined plane in the center, the position of the other end of the test piece is read on a scale. The push-out length (mm) of the test piece is taken as the stiffness.

According to the present invention, the stiffness of the support 10 is 40 mm-150 mm, preferably 60 mm-140 mm. A support having a stiffness of less than 40 mm is too soft to be inserted into the oral cavity easily. A support having a stiffness exceeding 150 mm is too hard and poor in followability to the irregular shape of the surface of the tongue or maxilla, easily causing foreign sensation during use. While the length of the long side of a sample is defined to be 150 mm in the 45° cantilever method, a sample exceeding 150 mm can be measured in effect by the cantilever shape test apparatus. Therefore, the stiffness of a sample having the stiffness exceeding 150 mm can be measured.

In the present invention, the material of the support 10 is not particularly limited only when the stiffness is within the above-mentioned range and the material can be used in the oral cavity, and, for example, a synthetic resin film, a cloth (non-woven fabric, woven fabric, knitted fabric etc.), a laminate of a synthetic resin film and a cloth and the like can be mentioned. As the synthetic resin film, for example, films produced using polyester such as polyethylene terephthalate and the like, polyamide such as nylon and the like, polyolefin such as polyethylene, polypropylene and the like, polyvinyl chloride, plasticized polyvinyl chloride, plasticized vinyl acetate-vinyl chloride copolymer, polyvinylidene chloride, ethylene-vinyl acetate copolymer, cellulose acetate, ethylcellulose, ethylene-ethyl acrylate copolymer, polytetrafluoroethylene, polyurethane and ionomer resin can be mentioned. As the cloth, for example, cloths produced using viscous rayon, copper ammonia rayon, diacetate, triacetate, promix, nylon, vinylon, vinylidene, polyvinyl chloride, polyester, acrylic, polyethylene, polypropylene, polyolefin, polyurethane, benzoate, polychlal and the like can be mentioned. As the laminate, laminates of the above-mentioned cloths on the above-mentioned synthetic resin films can be mentioned.

Of these, from the aspects of safety, economy, processability, texture and shape followability, cloths made from polyester or polyolefin fibers as a starting material, and laminates of such cloths on synthetic resin films are preferable. Particularly, by the use of the aforementioned laminate, a support having both fine texture and suitable stiffness can be obtained relatively easily. Particularly, it is preferable that the aforementioned laminate comprise a plaster layer on a cloth and does not comprise a plaster layer on a synthetic resin film, and when in use, the laminate be applied with a synthetic resin film surface in contact with the top surface of the tongue. By the use of a topical anesthetic in this manner, the taste buds sensing a bitter taste are covered by the synthetic resin film to a certain degree and the bitter taste of the topical anesthetic in the plaster layer is reduced.

When the support 10 is made of a synthetic resin film, its thickness is preferably 1 µm-200 µm, more preferably 2 µm-100 µm, from the aspects of handling property, processability, strength, shape followability and the like. A support having a thickness of not less than 1 µm is not damaged easily during production and when in use. A support having a thickness of not more than 200 µm does not become too stiff, and does not produce foreign sensation in the oral cavity when in use and does not injure the oral cavity with the corner.

When the support 10 is made of a cloth or a laminate of a cloth and a synthetic resin film, its thickness is preferably 100 µm-3000 µm, more preferably 200 µm-2000 µm, from the aspects of handling property, strength, texture, shape followability and the like. A support having a thickness of not less than 100 µm has sufficient strength, is easy to handle, and can be easily laminated on a synthetic resin film. A support having a thickness of not more than 3000 µm does not produce foreign sensation easily in the oral cavity when in use and shows fine processability.

When the support 10 is made of a cloth, its mass per unit area is preferably 30-1000 g/m², more preferably 50-800 g/m² from the aspects of strength, handling property and toughness. When the mass per unit area is not less than 30 g/m², the strength becomes sufficient, and handling and lamination on a film become easy. When the mass per unit area is not more than 1000 g/m², the support has suitable flexibility, easily follows the shape of the oral cavity and does not produce foreign sensation easily.

In consideration of the application site and use, the support 10 preferably has an about rectangular shape. In consideration of the shape of the mouth of human and the oral cavity, the length in the shorter direction of the support is preferably 10-60 mm, more preferably 20-50 mm. The length in the longitudinal direction of the support is preferably 50-120 mm, more preferably 60-110 mm.

The plaster layer 20 contains at least a topical anesthetic and an adhesive polymer and is formed on one surface or both surfaces of one end in the longitudinal direction of the support 10. In the embodiment of Fig. 1, the plaster layer 20 is formed only on one surface.

The topical anesthetic to be contained in the plaster layer 20 is specifically one or more selected from cocaine, procaine, chloroprocaine, tetracaine, benzocaine, lidocain, mepivacaine, prilocaine, bupivacaine, dibucaine, propoxycaine, etidocaine, dyclonine and pharmacologically acceptable salts thereof. The content of each of the selected drugs in the plaster layer 20 can be appropriately determined depending on the thickness and area of the plaster layer 20, and is generally about 1-80 wt%, preferably about 2-70 wt%. When the content of the topical anesthetic is not less than 1 wt%, release of the drug in an amount effective for topical anesthesia is expected and when it is not more than 80 wt%, the possibility of plaster layer 20 falling from the support 10 is low and waste of topical anesthetic becomes less, which is economically preferable.

The adhesive polymer to be contained in the plaster layer 20 is not particularly limited as long as it retains the topical anesthetic selected in the above, is capable of closely or weakly adhering to the maxilla of oral cavity and tongue, and suitably releasing the topical anesthetic in the oral cavity. The topical anesthetic to be retained may have been dissolved, supersaturated, crystallized, dispersed and the like. Preferably, the adhesive polymer satisfies at least one of "substantially free of dissolution in water" and "substantially free of water absorption", because the adhesive polymer shows suitable adhesiveness, drug releasability, and shape retention property in the oral cavity. Here, "substantially free of dissolution in water" means solubility of not more than 5 wt% in a sufficient amount of water at 20°C, and pressure sensitive adhesiveness at normal temperature. The "substantially free of water absorption" means absorption amount of not more than 5 wt% of a sufficient amount of water at 20°C, and pressure sensitive adhesiveness at normal temperature.

Here, as an adhesive polymer satisfying at least one of the above-mentioned "substantially free of dissolution in water" and "substantially free of water absorption", an adhesive polymer of an acrylic polymer; rubber adhesive polymers such as a styrene-isoprene-styrene block copolymer, a styrene-butadiene-styrene block copolymer, polyisoprene, polybutadiene and the like; silicone adhesive polymers such as silicone rubber, dimethylsiloxane base, diphenylsiloxane base and the like; adhesive polymers such as polyvinylmethyl ether, polyvinylethyl ether, polyvinylisobutyl ether and the like; vinyl ester adhesive polymers such as vinyl acetate-ethylene copolymer and the like; polyester adhesive polymers comprising carboxylic acid components such as dimethyl terephthalate, dimethyl isophthalate, dimethyl phthalate and the like and polyhydric alcohol components such as ethylene glycol and the like; and the like can be specifically mentioned. Of these, an adhesive polymer comprising an acrylic polymer is preferable from anchor property to support 10, suitable adhesion to mucous membrane, drug stability and the like.

The plaster layer 20 may contain various additives as necessary. As the additives, flavoring, sweetener, plasticizer, absorption promoter and the like can be mentioned. In consideration of the reduction of bitter taste due to the topical anesthetic, addition of flavoring and sweetener is particularly preferable. As the flavoring, for example, natural flavoring and synthetic flavoring such as mints such as peppermint, spearmint; menthol, artificial vanilla, cinnamon, orange, lemon, banana, apple, pear, blueberry, strawberry, cherry, grape and the like; and the like can be mentioned. As the sweetener, for example, sugars such as sucrose, glucose, dextrose, fructose and the like; amino acids and dipeptide sweeteners such as saccharin and a salt thereof, cyclamic acid and a salt thereof, aspartam and the like; sugar alcohols such as dihydrochalcone compound, sucralose, glycyrrhizin, stevioside, rebaudioside, sorbitol, mannitol, xylitol and the like; and the like can be mentioned. Where necessary, flavoring and sweetener may be added to the support 10 itself by a known method.

When a plasticizer is added to the plaster layer 20, an adhesive polymer is plasticized and adhesive power to the upper surface of the tongue, hard palate or soft palate can be adjusted. As the plasticizer, for example, diisopropyl adipate, diacetyl sepagate and the like can be mentioned, and one or more kinds thereof can be used.

The plaster layer 20 may contain an absorption promoter. The absorption promoter is a compound having a function to improve solubility and diffusion property of the topical anesthetic in an adhesive polymer of the plaster layer 20, and the following compounds can be concretely mentioned. As a compound that mainly enhances drug solubility, for example, glycols such as ethylene glycol, diethylene glycol, propylene glycol, triethylene glycol, polyethylene glycol, polypropylene glycol and the like can be mentioned. As a compound that mainly enhances drug diffusion property, fats and oils such as olive oil, castor oil, squalene, lanolin and the like; and the like can be mentioned. Other than these, hydrocarbons such as liquid paraffin; various surfactants; glycerol monoesters such as ethoxylated stearyl alcohol, oleic acid monoglyceride, caprylic acid monoglyceride and lauryl acid monoglyceride; glycerol diester, glycerol triester or a mixture thereof; higher fatty acid esters such as ethyl laurate, isopropyl myristate, isotridecyl myristate, octyl palmitate, isopropyl palmitate, ethyl oleate and diisopropyl adipate; higher fatty acid such as oleic acid and caprylic acid; N-methylpyrrolidone, 1,3-butanediol and the like can be mentioned.

Where necessary, a small amount of ascorbic acid, citric acid and the like may be added to the plaster layer 20 to promote secretion of saliva.

According to the present invention, the drug release of the topical anesthetic in the oral cavity can be adjusted in a wide range depending on the kind and composition ratio of an adhesive polymer and an additive contained in the plaster layer 20, which in turn affords use of a topical anesthetic at an extremely high utilization rate.

The drug utilization rate when lidocain hydrochloride 2%-containing viscous agent (3 mL) conventionally used is spat out the oral cavity after 3 min is about 5-15%, and most of the drug is wasted without being used. The drug utilization rate is calculated from the residual amount. The drug utilization rate when the topical anesthetic preparation of the present invention is administered to the oral cavity for 3 min in the same manner as in the above is extremely high and about 60-90%. As evidenced, the topical anesthetic preparation of the present invention affords a sufficient anesthetic effect by a small amount of the drug as compared to conventional cases. Therefore, even if the preparation is taken by mistake, it is highly safe because the amount of the drug is small. In addition, the amount of harmful chemical remaining in the preparation after use, and the medical cost necessary for the topical anesthetic can be reduced.

A pump spray type surface anesthetic often used for anesthetizing throat has a high drug utilization rate. However, precise administration of a pump spray type surface anesthetic to the lesion is difficult, a nozzle tube may be clogged, and there is also the possibility of infection due to the sharing thereof among patients. The topical anesthetic preparation of the present invention is almost free of such fears.

The shape of the support 10 on which to form a plaster layer 20 is not particularly limited. In view of usability and to minimize the stimulation to the throat, one end where the plaster layer 20 is formed is preferably curved into a circular arc, which is more specifically, for example, an about semicircle. In this case, the plaster layer 20 is formed only in the circular arc of the support 10, or formed bridging the circular arc and a part of the rectangle following the circular arc, where the size in the shorter direction is constant.

When the shape of one end of the support 10 is an about semicircle and the plaster layer 20 is formed on the semicircle, the radius of the about semicircle is preferably 5-30 mm, more preferably 10-25 mm. When the plaster layer 20 is formed bridging the about semicircle and a part of the rectangular part following the about semicircle, the radius of the about semicircle is preferably 5-30 mm, more preferably 10-25 mm. In this event, the part of the rectangle, where the plaster layer 20 is formed, is from the connection between the about semicircle and the rectangle to preferably 2-30 mm, more preferably 5-20 mm, toward the center of the support 10.

The area of the part where the plaster layer 20 is formed is preferably 1-45 cm², more preferably 4-35 cm².

According to the present invention, the plaster layer 20 containing a topical anesthetic may be formed only on one surface or may be formed on both surfaces of one end of the support 10. When the layer is formed on the both surfaces, the dose can be increased without changing the concentration of the topical anesthetic. When the plaster layer 20 is formed on the both surfaces of the support 10, the shape, size and composition of the plaster layer 20 formed on the respective surfaces may be the same or different.

For example, a plaster layer containing a topical anesthetic is formed on one surface of the support 10, and a plaster layer containing a flavoring and a sweetener to reduce a bitter taste of the topical anesthetic may be formed on the other surface.

The surface of the plaster layer 20 is protected by covering with a release liner, so that the surface of the plaster layer 20 is not exposed until preferably immediately before use, for the purpose of preventing undesired adhesion of the plaster layer 20 to instruments, containers and the like during production, transport or preservation, and preventing degradation of topical anesthetic and the like. In this case, when in use, the release liner is peeled off to expose the surface of the plaster layer 20 and then the preparation is inserted into the oral cavity. The release liner is not particularly limited as long as it can be easily peeled off from the plaster layer 20 when in use, and, for example, films of polyester, polyvinyl chloride, polyvinylidene chloride, polyethylene terephthalate and the like; paper such as fine paper, glassine paper and the like; or laminate films of fine paper or glassine paper and polyolefin film and the like, which have undergone release treatment by applying silicone resin, fluororesin and the like to the surface to come into contact with the plaster layer 20, can be mentioned.

The thickness of the release liner is preferably 10-200 µm, more preferably 25-100 µm.

In the topical anesthetic preparation of the present invention, gripper 30 is preferably formed on the other end of the support. The gripper 30 is held by a hand (fingers) of the person using the preparation and the like and functions to facilitate insertion of one end of the support 10 of the preparation into the desired site in the oral cavity. Since the gripper 30 is outside the mouth while the preparation is inserted into the oral cavity for topical anesthesia, it can be visually identified from the outside. Of the ends in the longitudinal direction of the support, the other end of the support is the opposite end from the end where the plaster layer is formed.

Since the topical anesthetic preparation of the present invention has a gripper, the topical anesthetic preparation (more specifically the support) is long enough to stick out the other end of the support from the mouth when the plaster layer is in the oral cavity. In consideration of the presence of a gripper, the specific length of the support in the longitudinal direction is preferably 50-120 mm, more preferably 60-110 mm.

The shape of the gripper 30 and the like are not particularly limited as long as it can be easily held when in use, thus facilitating smooth insertion of the preparation into the oral cavity, it can be easily pulled out from the oral cavity on termination of the anesthesia, and the use of the preparation can be identified from the outside during use. The other end of the support 10 may be used as the gripper 30 without modification. In this event, the other end is preferably is treated for direct printing of letters, numbers, figures or color for identification. Alternatively, a gripper may be formed by laminating a film or sheet carrying printed letters, numbers, figures or color for identification, on the other end of the support 10. As shown in Fig. 2, the gripper 30 may indicate, for example, letters, symbols, numbers, use, name of drug, dose, drug concentration, cautions and the like, and dose and administration frequency of the drug may be distinguished by the whole color tone.

When the gripper 30 is directly printed on the support 10, conventional printing methods can be used as the method of printing on the support 10. For example, gravure coating, roll coating, knife coating, spray coating, gravure printing, flexographic printing, screen printing, offset printing and the like can be mentioned.

When a film or sheet is laminated on the support 10, dry laminate adhesion, heat sealing and the like can be used for adhesion thereof to the support 10. The material of the film or sheet to be used for the gripper 30 is not particularly limited as long as the gripper can be held during insertion into the oral cavity, and polyester or polyolefin is preferably used in view of safety, economic aspect, processability and operability. Moreover, the shape thereof is not particularly limited and may be an about square, an about rectangle, an about semicircle, an about semiellipse and the like. In view of the processability and identifiability from the plaster layer 20, the shape is preferably an about square or an about rectangle. When the gripper 30 is an about square, one side is preferably 10 - 60 mm, more preferably 20 - 50 mm. When the gripper 30 is an about rectangle, the longer direction of the about rectangle is preferably in the same direction as the shorter direction of the support 10, and the long side is preferably 10 - 60 mm, more preferably 20 - 50 mm. In addition, the short side of the rectangle is preferably 10 - 40 mm, more preferably 15 - 30 mm,

The thickness of the film or sheet to be used for the gripper 30 is appropriately determined according to the property of the material, shape and area, and is preferably 5-1000 µm, more preferably 10-500 µm. The angle of the gripper 30 may have a circular arc having a suitable radius of curvature so as to prevent scratching the skin or mucous membrane during insertion into the oral cavity or during anesthesia. The gripper 30 may have a concave, slit or gap to improve operability during insertion and withdrawal thereof.

The topical anesthetic preparation of the present invention may be sterilized as necessary by gas, electron beam or radiation generally known and the like after being packaged with a microorganism impermeable material.

The topical anesthetic preparation of the present invention is generally inserted into the oral cavity with the plaster layer 20 containing the topical anesthetic facing upward and in contact with the palate, i.e., hard palate or soft palate, so as to avoid a bitter taste of the topical anesthetic and the like. The insertion is stopped when one end of the support 10 has reached the suitable position of the soft palate. The subject stands or sits still with the mouth shut in this state for 30 seconds to 5 minutes, preferably 1 to 4 minutes. As a result, an anesthesia transmission effect in the mucous membrane and a diffusion effect by exuded drug into the saliva are expressed, and the pharyngeal reflex during insertion of an endoscope can be suppressed. Particularly, a higher anesthetic effect can be obtained by facing upward as in gargling. According to the preferable topical anesthetic preparation of the present invention, since gripper 30 is exposed from the mouth cavity during use, the treatment condition can be easily understood, and medical procedure, drug name, dose, drug concentration, treatment frequency and the like can be easily identified from the description on and color tone of the gripper 30.

The topical anesthetic preparation of the present invention may constitute a commercial package together with a written matter stating that the preparation can or should be used for an intraoral pretreatment for upper gastrointestinal endoscopy so as to suppress the pharyngeal reflex during the above-mentioned endoscopy.

### Examples

The present invention is explained in detail by referring to examples. In the following description, "parts" means "parts by weight". The stiffness was measured as described in the foregoing description.

### (Example 1)

A polyethylene terephthalate film (thickness 6 µm) was coated with a mixed solution of a polyester urethanepolyol resin (72 parts), aromatic polyisocyanate (8 parts) and ethyl acetate (240 parts) and dried to form a dry laminate adhesive layer having a thickness of 10 µm. A spunlace non-woven fabric (mass 90 g/m², thickness about 420 µm, stiffness 115 mm) prepared from a composite fiber comprising 50% polyethylene terephthalate and 50% polypropylene having an about triangle section having a bottom side of 5 µm and height 10 µm was laminated on this adhesive layer. Then, this laminate was cut into a rectangle (short side 30 mm, long side 80 mm) to give a support sheet 10. Separately, a mixed solution of acrylic acid/octyl acrylate copolymer (40 parts) and lidocain (60 parts) in ethyl acetate was coated on a 75 µm thick polyethylene terephthalate film that had undergone a silicon release treatment in an amount to achieve 31 g/m² after drying and dried to give a plaster sheet layer 20. The plaster layer 20 was cut out in a semicircle having a radius of 15 mm, and laminated on the surface of a non-woven fabric on one end of the support 10 with the circular arc facing outside. The corner of the support was cut off along the shape of the circular arc of the plaster layer 20. A polyethylene terephthalate film (length 20 mm, width 30 mm, thickness 50 µm), on which drug name, dose of drug and use were printed in white letters on a blue base, was laminated on the surface of a non-woven fabric on the other end of the support 10 by a method similar to the above-mentioned dry lamination to give a gripper 30. A plane view of the thus-obtained topical anesthetic preparation of the present invention is shown in Fig. 2.

### (Example 2)

In the same manner as in Example 1, a rectangular support sheet 10 (short side 20 mm, long side 80 mm) was obtained. A mixed solution of acrylic acid/octyl acrylate copolymer (40 parts) and lidocain (60 parts) in ethyl acetate was coated on a 75 µm thick polyethylene terephthalate film that had undergone a silicon release treatment in an amount to achieve 70 g/m² after drying and dried to give a plaster sheet layer 20. This plaster layer 20 was cut out in a semicircle having a radius of 10 mm, and laminated on the surface of a non-woven fabric on one end of the support 10 with the circular arc facing outside. The corner of the support was cut off along the shape of the circular arc of the plaster layer 20. A polyethylene terephthalate film (length 20 mm, width 30 mm, thickness 50 µm), on which drug name, dose of drug and use were printed in white letters on a green base by gravure printing, was laminated on the surface of a non-woven fabric on the other end of the support 10 by a method similar to the above-mentioned dry lamination to give a gripper 30. The topical anesthetic preparation of the present invention was obtained in this way.

### (Example 3)

In the same manner as in Example 1, a rectangular support sheet 10 (short side 40 mm, long side 100 mm) was obtained. A mixed solution of acrylic acid/octyl acrylate copolymer (40 parts) and lidocain (60 parts) in ethyl acetate was coated on a 75 µm thick polyethylene terephthalate film that had undergone a silicon release treatment in an amount to achieve 18 g/m² after drying and dried to give a plaster sheet layer 20. This plaster layer 20 was cut out in a semicircle having a radius of 20 mm, and laminated on the surface of a non-woven fabric on one end of the support 10 with the circular arc facing outside. The corner of the support was cut off along the shape of the circular arc of the plaster layer 20. A polyethylene terephthalate film (length 20 mm, width 40 mm, thickness 50 µm), on which drug name, dose of drug and use were printed in white letters on an orange base, was laminated on the surface of a non-woven fabric on the other end of the support 10 by a method similar to the above-mentioned dry lamination to give a gripper 30. The topical anesthetic preparation of the present invention was obtained in this way.

### (Example 4)

In the same manner as in Example 1, a rectangular support sheet 10 (short side 30 mm, long side 100 mm) was obtained. A mixed solution of acrylic acid/octyl acrylate copolymer (40 parts) and lidocain (60 parts) in ethyl acetate was coated on a 75 µm thick polyethylene terephthalate film that had undergone a silicon release treatment, in an amount to achieve 31 g/m² after drying and dried to give a plaster sheet layer (1). This plaster layer (1) was cut out in a semicircle having a radius of 15 mm, and laminated on the surface of a non-woven fabric on one end of the support 10 with the circular arc facing outside. Then, a dispersion mixture of acrylic acid/octyl acrylate copolymer (40 parts), polyvinyl ester copolymer (40 parts), polyvinylpyrrolidone (55 parts), stevioside (sweetener, 4 parts), vanillin (flavor, 1 part) in ethyl acetate was coated on a 75 µm thick polyethylene terephthalate film that had undergone a silicon release treatment, in an amount to achieve 10 g/m² after drying and dried to give a plaster layer (2). This plaster layer (2) was cut out in a sequential shape of a 30 mm square and a semicircle having a radius of 15 mm, and adhered to a surface facing the plaster layer (1) of the support 10, namely, on the surface of a polyethylene terephthalate film, with the circular arc on the outside. The corner of the support was cut off along the shape of the circular arc of the plaster layer (1) and plaster layer (2). A polyethylene terephthalate film (length 20 mm, width 30 mm, thickness 50 µm), on which drug name, dose of drug and use were printed in white letters on a purple base, was laminated on the surface of a non-woven fabric on the other end of the support 10 by a method similar to the above-mentioned dry lamination to give a gripper 30. The topical anesthetic preparation of the present invention was obtained in this way.

### (Comparative Example 1)

A circular knitted fabric (mass 98 g/m², thickness about 400 µm, stiffness 15 mm) prepared from a polyester fiber having a fiber diameter of 20 µm and a circular section was cut into a rectangle (short side 30 mm, long side 80 mm) to give a support sheet. Then, a mixed solution of acrylic acid/octyl acrylate copolymer (40 parts) and lidocain (60 parts) in ethyl acetate was coated on a 75 µm thick polyethylene terephthalate film that had undergone a silicon release treatment in an amount to achieve 31 g/m² after drying and dried to give a plaster sheet layer. This plaster layer was cut out in a semicircle having a radius of 15 mm, and laminated on the surface of the circular knitted fabric on one end of the support with the circular arc facing outside. The corner of the support was cut off along the shape of the circular arc of the plaster layer. A polyethylene terephthalate film (length 20 mm, width 30 mm, thickness 50 µm), on which drug name, dose of drug and use were printed in white letters on a blue base, was laminated on the surface of the circular knitted fabric on the other end of the support by a method similar to the above-mentioned dry lamination to give a gripper. In this manner, a topical anesthetic preparation was obtained.

### (Comparative Example 2)

A non-woven fabric (stiffness 170 mm, mass 185 g/m², thickness about 2.3 mm) made of rayon acrylic binder was cut into a rectangle (short side 30 mm, long side 80 mm) to give a support sheet. Then, a mixed solution of acrylic acid/octyl acrylate copolymer (40 parts) and lidocain (60 parts) in ethyl acetate was coated on a 75 µm thick polyethylene terephthalate film that had undergone a silicon release treatment in an amount to achieve 31 g/m² after drying and dried to give a plaster sheet layer. This plaster layer was cut out in a semicircle having a radius of 15 mm, and laminated on the surface of a non-woven fabric on one end of the support with the circular arc facing outside. The corner of the support was cut off along the shape of the circular arc of the plaster layer. A polyethylene terephthalate film (length 20 mm, width 30 mm, thickness 50 µm), on which drug name, dose of drug and use were printed in white letters on a blue base, was laminated on the surface of a non-woven fabric on the other end of the support by a method similar to the above-mentioned dry lamination to give a gripper 30. In this manner, a topical anesthetic preparation was obtained.

### (Comparative Example 3)

A viscous agent preparation for surface anesthesia, which contained 2% lidocain hydrochloride was used for comparison.

The preparations obtained in the above-mentioned Examples 1, 2 and 3 and Comparative Examples 1, 2 and 3 were subjected to a practicality test by volunteers. In the test, the preparations of Examples 1, 2 and 3 and Comparative Examples 1 and 2 were inserted until the tip of the plaster layer 20 reached the soft palate in the oral cavity of the volunteers, maintained for 3 min. and then removed. The operability, identifiability during administration, foreign sensation during administration, bitter taste during administration, drug utilization rate in the test and anesthesia after administration were evaluated. In addition, the preparation (3 mL) of Comparative Example 3 was injected with a plastic syringe into the oral cavity of the volunteers, maintained for 3 min and removed. The operability, identifiability during administration, foreign sensation during administration, bitter taste during administration, drug utilization rate in the test and anesthesia after administration were evaluated. The results are shown in Table 1.

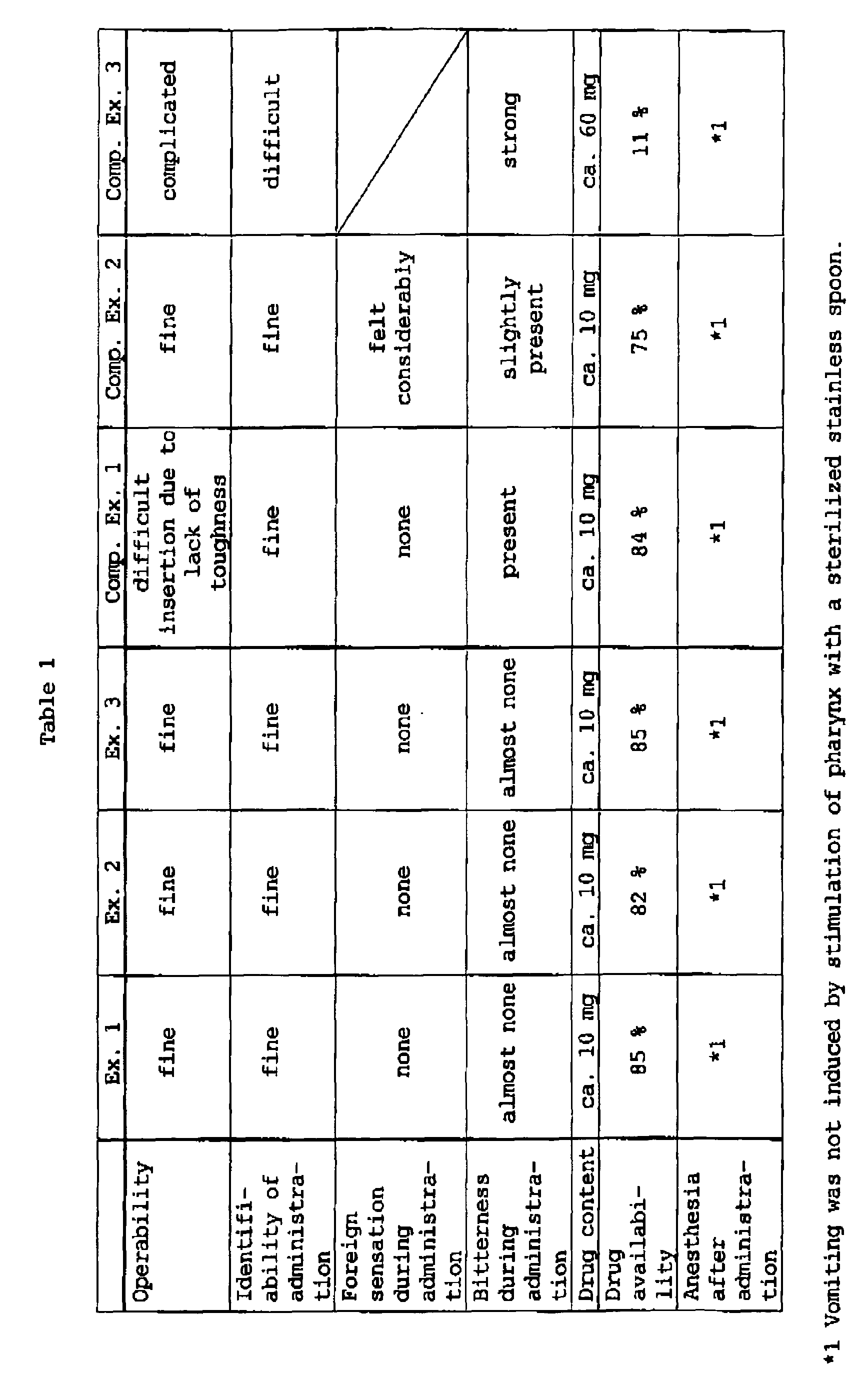

As shown in Table 1, Examples 1-3 showed fine operability and identifiability, posed no problem with regard to foreign sensation and bitter taste during administration, and drug utilization rate was high. In contrast, Comparative Examples 1-3 were associated with a problem in some of the plural items of operability and identifiability.

The topical anesthetic preparation of the present invention is used to suppress the pharyngeal reflex during insertion of an endoscope into the pharynx by topically anesthetizing the pharynx as a pretreatment for upper gastrointestinal endoscopy, permits easy identification of the use or non-use, uses a topical anesthetic at a high efficiency, and can be preferably used in clinical situations.

## Claims

1. A topical anesthetic preparation for a pretreatment for upper gastrointestinal endoscopy, which comprises
- a long support (10) having a stiffness of 40-150 mm (as measured according to JIS L 1085, 1992, 5.7, Method A),
- a plaster layer (20) containing at least a topical anesthetic and an adhesive polymer, which is formed on one surface or both surfaces of one end in the longitudinal direction of the support (10); and
- a gripper (30) formed on the other end in the longitudinal direction of the support (10), wherein the gripper (30) has a constitution such that it can be held to contact the plaster layer (20) with an intraoral part where the topical anesthetic is allowed to act on.

2. The topical anesthetic preparation of claim 1, wherein the gripper (30) is printed to allow the identification

3. The topical anesthetic preparation of claim 1, wherein the material of the support (10) is selected from the group consisting of a synthetic resin film, a cloth and a laminate of a cloth and a synthetic resin film.

4. The topical anesthetic preparation of claim 1, wherein the plaster layer (20) is fromed in the circular arc of the support (10).

5. The topical anesthetic preparation of claim 1 or 2, wherein the gripper (30) is formed by direct printing on the support (10).

6. The topical anesthetic preparation of claim 1 or 2, wherein the gripper (30) is formed by laminating a film or sheet after a printing treatment on the support (10).

## Patentansprüche

1. Topisches Anästhesiepräparat zur Vorbehandlung für die obere gastrointestinale Endoskopie, umfassend:
- einen langen Träger (10) mit einer Steifigkeit von 40 bis 150 mm (gemessen gemäß JIS L 1085, 1992, 5.7, Methode A);
- eine Pflasterschicht (20), die wenigstens ein topisches Anästhetikum und ein adhäsives Polymer enthält und auf einer Oberfläche oder beiden Oberflächen eines Endes in Längsrichtung des Trägers (10) ausgebildet ist; und
- einen Greifer (30), der am anderen Ende in Längsrichtung des Trägers (10) ausgebildet ist, wobei der Greifer (30) eine Konstitution hat, aufgrund der er so gehalten werden kann, dass er die Pflasterschicht (20) mit einem intraoralen Teil in Kontakt bringt, wo man das topische Anästhetikum einwirken lässt.

2. Topisches Anästhesiepräparat gemäß Anspruch 1, wobei der Greifer (30) so bedruckt ist, dass eine Identifizierung ermöglicht wird.

3. Topisches Anästhesiepräparat gemäß Anspruch 1, wobei das Material des Trägers (10) aus der Gruppe ausgewählt ist, die aus einer Kunstharzfolie, einem Textilstoff und einem Laminat aus einem Textilstoff und einer Kunstharzfolie besteht.

4. Topisches Anästhesiepräparat gemäß Anspruch 1, wobei die Pflasterschicht (20) im Kreisbogen des Trägers (10) ausgebildet ist.

5. Topisches Anästhesiepräparat gemäß Anspruch 1 oder 2, wobei der Greifer (30) durch direktes Aufdrucken auf den Träger (10) ausgebildet ist.

6. Topisches Anästhesiepräparat gemäß Anspruch 1 oder 2, wobei der Greifer (30) durch Laminieren eines Films oder einer Folie nach einer Bedruckung des Trägers (10) ausgebildet ist.

## Revendications

1. Préparation d'anesthésique topique pour un prétraitement en endoscopie gastro-intestinale supérieure, qui comprend :
■ un support long (10) ayant une dureté de 40 à 150 mm (telle que mesurée selon JIS L 1085, 1992, 5.7, méthode A),
■ une couche de plâtre (20) contenant au moins un anesthétique topique et un polymère adhésif, qui est formée sur une surface ou sur les deux surfaces d'une extrémité dans le sens longitudinal du support (10) ; et
■ une partie de préhension (30) formée sur l'autre extrémité dans le sens longitudinal du support (10), la partie de préhension (30) ayant une constitution telle qu'elle peut être tenue pour mettre en contact la couche de plâtre (20) avec une partie intra-buccale sur laquelle l'anesthésique topique peut agir.

2. Préparation d'anesthésique topique selon la revendication 1, dans laquelle la partie de préhension (30) porte une impression pour permettre son identification.

3. Préparation d'anesthésique topique selon la revendication 1, dans laquelle le matériau du support (10) est choisi dans le groupe constitué par un film en résine synthétique, une toile et un stratifié d'une toile et d'un film en résine synthétique.

4. Préparation d'anesthésique topique selon la revendication 1, dans laquelle la couche de plâtre (20) est formée dans l'arc de cercle du support (10).

5. Préparation d'anesthésique topique selon la revendication 1 ou 2, dans laquelle la partie de préhension (30) est formée par impression directe sur le support (10).

6. Préparation d'anesthésique topique selon la revendication 1 ou 2, dans laquelle la partie de préhension (30) est formée par stratification d'un film ou d'une feuille après un traitement d'impression sur le support (10).
